# EUROPEAN PATENT SPECIFICATION

(11) **EP 3 008 049 B1**
(45) Date of publication and mention of the grant of the patent: **10.01.2018**
(21) Application number: 13886834.4
(22) Date of filing: 14.06.2013
(51) Int. Cl.: C07D 307/52, C07F 15/00

(54) **PROCESS FOR THE PRODUCTION OF FURANIC COMPOUNDS COMPRISING AT LEAST ONE AMINE FUNCTION**
VERFAHREN ZUR HERSTELLUNG VON FURANVERBINDUNGEN MIT MINDESTENS EINER AMINFUNKTION
PROCÉDÉ POUR LA PRODUCTION DE COMPOSÉS FURANIQUES COMPRENANT AU MOINS UNE FONCTION AMINE

(43) Date of publication of application: 20.04.2016
(73) Proprietor: Rhodia Operations, 75009 Paris (FR)
(72) Inventor: DECAMPO, Floryan, Shanghai 201108 (CN); LI, Peng, Shanghai 201108 (CN)
(74) Representative: Benvenuti, Federica
(86) International application number: PCT/CN2013/077240
(87) International publication number: WO 2014/198057

(56) References cited:
- ON-YI LEE, KA-LUN LAW AND DAN YANG: "Secondary Amine Formation from Reductive Amination of Carbonyl Compounds Promoted by Lewis Acid Using the InCl3/Et3SiH System", ORGANIC LETTERS, vol. 11, no. 15, 10 July 2009 (2009-07-10) , pages 3302-3305, XP002762160, DOI: 10.1021/ol901111g
- ANNEGRET TILLACK, DIRK HOLLMANN, DIRK MICHALIK, MATTHIAS BELLER: "A novel ruthenium-catalyzed amination of primary and secondary alcohols", TETRAHEDRON LETTERS, vol. 47, no. 50, 11 December 2006 (2006-12-11), pages 8881-8885, XP002762161, DOI: 10.1016/j.tetlet.2006.10.042
- KAWAHARA, RYOKO ET AL.: 'Multialkylation of Aqueous Ammonia with Alcohols Catalyzed by Water-Soluble Cp*Ir-Ammine Complexes' JOURNAL OF THE AMERICAN CHEMICAL SOCIETY vol. 132, no. 43, 2010, pages 15108 - 15111, XP055074773
- NORINDER, JAKOB ET AL.: 'Highly Efficient and Selective Catalytic N-Alkylation of Amines with Alcohols in Water' CHEMCATCHEM vol. 3, no. 9, 2011, pages 1407 - 1409, XP055055913
- KAWAHARA, RYOKO ET AL.: 'N-Alkylation of Amines with Alcohols Catalyzed by a Water-Soluble Cp*Iridium Complex: An Efficient Method for the Synthesis of Amines in Aqueous Media' ADVANCED SYNTHESIS & CATALYSIS vol. 353, no. 7, 2011, pages 1161 - 1168, XP055055911

## Description

The present invention concerns a process for the production of furanic compound comprising at least one amine function, comprising reacting a furanic compound having at least one hydroxyl function or at least one aldehyde function with a second reactant having an amine function, in the presence of an iridium catalyst.

### PRIOR ART

Amines are of significant importance for the chemical industry, but also for numerous biological processes. For instance, amino acids and nucleotides constitute essential biological building blocks and numerous bioactive compounds such as vitamins, hormones, alkaloids, neurotransmitters, or natural toxics contain amino groups. It is, therefore, not surprising, that numerous amines and their derivatives find application as agrochemicals, pharmaceuticals, or food additives. Several million tons of amines are produced annually. They are widely used in both the bulk and fine chemical industries as fundamental materials, additives, dyes, and agrochemicals.

The most common strategies to produce secondary or tertiary amines involve treating a primary amine with an alkylating reagent having a good leaving group, such as RX with X=halide, OTs or OTf. The main drawback of this conventional approach is the generation of a stoichiometric amount of wasteful (in)organic salts, as well as low secondary/tertiary amine product selectivity.

In recent years, *N*-monoalkylation was demonstrated, wherein an alcohol is used in place of RX and transition metal catalysis involves Ru, Ir, Cu, or Ag catalyst precursors. M. H. S. A. Hamid, P. A. Slatford, J. M. J. Williams, Adv. Synth. Catal. 2007, 349, 1555; T. D. Nixon, M. K. Whittlesey, J. M. J. Williams, Dalton Trans. 2009, 753; G. Guillena, D. J. Ramón, M. Yus, Chem. Rev. 2010, 110, 1611; G. E. Dobereiner, R. H. Crabtree, Chem. Rev. 2010, 110, 681. S. Bähn, S. Imm, L. Neubert, M. Zhang, H. Neumann, M. Beller, ChemCatChem 2011, 3, 1853. These transition metal-catalyzed *N*-alkylations are usually redox-type reactions involving a "borrowing hydrogen" or "hydrogen autotransfer" mechanism (alcohol oxidation/imine formation/imine hydrogenation).

Although these reactions have found many applications using benzylic-type and saturated alcohols as the *N*-alkylating agents, the supposedly generated metal hydride species are incompatible with some functional groups, including olefins.

A novel straightforward method of *N*-alkylation using iron catalysis, such as FeBr₃, which involves a substitution (S_{N}) at the sp³-carbon atom bearing the hydroxy group of the alcohol was also described in 2011 (Y. Zhao, S. Wan Foo, S. Saito, Angew. Chem. Int. Ed. 2011, 50, 3006).

Specifically, Al(OTf)₃ was recently used as a catalyst for direct amination of conjugated allylic alcohols and benzylic type alcohols. It appears however that this proposed catalyst leads to a non sufficient conversion of however that this proposed catalyst leads to a non sufficient conversion of the aniline plus benzyl alcohol reaction (K. Mashima et al., Adv. Synt. Catal. 2012, 354, 2447).

It also appears that while the direct amination of benzylic type alcohols is occurring with high yields and conversions, same reactions with less reactive alcohols such as furanic compounds are less effective and lead to a drop of the yields even with the use of complex catalysts such as the combination of [Ru₃(CO)₁₂] and CataCXiumPCy (C. Gonzales-Arellano et al., Green. Chem., 2010, 12, 1281-1287).

As regards the technical background of the instant invention, reference may also be made to the following documents :
▪ Organic Letters, viol.11, no.15 (2009-07-10) p. 3302-3305
▪ Tetrahedron Letters, vol. 47, no. 50 (2006-12-11) p. 8881-8885
▪ Journal of the American Society, vol. 132, no. 43 (2010) p. 1407-1409
▪ ChemCatChem, vol. 3, no. 9 (2011) p. 1407-1409
▪ Advanced Synthesis and Catalysis, vol. 353, no.7 (2011) p. 1161-1168

It exists then a need to provide new catalysts to produce different amines by direct amination of alcohols with a sufficient yield, high conversion and an improved reaction selectivity, notably permitting then to produce amines by shifting from conventional petrochemical feedstocks towards biomass-based feedstocks as the furanic compounds, 5-Hydroxymethylfurfural (HMF) for instance.

### INVENTION

It appears now that it is perfectly possible to carry out a process to lead a N-alkylation of a furanic compound by using a specific amine and a specific iridium catalyst, to notably obtain sufficient yields and conversions in comparison with the process involving furanic compounds in the prior art.

The present invention then concerns a process for the production of furanic compound comprising at least one amine function, comprising reacting:
- A first reactant being a furanic compound having at least one hydroxyl function or at least one aldehyde function, with
- A second reactant being a compound of formula (I),

   R-CH₂-NH₂ (I)

   in the presence of a catalyst of formula (II) or (III):

   [CpIrX₂]₂ (II)

   [CpIr(NH₃)₃][X]₂ (III)

   and optionally in the presence of a reductant agent;
   wherein:
   - R is H or a straight, branched or cyclic hydrocarbon group;
   - X is a halide, such as Br, I, or Cl;
   - Cp is a cyclopentadienyl group, which may be optionally substituted by from 1 to 5 independently selected hydrocarbyl substituents, such as methyl, ethyl or propyl.

Without being particularly bound by the theory, according to the reaction process of the invention, N-alkylation occurs by dehydrogenation of the hydroxyl function to aldehyde, and subsequently imine is formed through dehydrative condensation between an amine (I) and the aldehyde intermediate, followed by transfer hydrogenation with the transiently formed "Ir-H" species. In case of substrates with aldehyde groups, an external reductant like a secondary alcohol may be required for transfer hydrogenation.

### DEFINITIONS

"Alkyl" as used herein means a straight chain or branched saturated aliphatic hydrocarbon. Preferably alkyl group comprises 1-18 carbon atoms. Representative saturated straight chain alkyls include methyl, ethyl, n-propyl, n-butyl, n-pentyl, and the like; while saturated branched alkyls include isopropyl, sec-butyl, isobutyl, tert-butyl, isopentyl, and the like.

"Alkenyl", as used herein, refers to an aliphatic group containing at least one double bond and is intended to include both "unsubstituted alkenyls" and "substituted alkenyls", the latter of which refers to alkenyl moieties having substituents replacing a hydrogen on one or more carbon atoms of the alkenyl group. Representative unsaturated straight chain alkenyls include ethenyl, propenyl, butenyl, pentenyl, hexenyl, heptenyl, octenyl, nonenyl, decenyl and the like.

The term "cyclic group" means a closed ring hydrocarbon group that is classified as an alicyclic group, aromatic group, or heterocyclic group. The term "alicyclic group" means a cyclic hydrocarbon group having properties resembling those of aliphatic groups.

"Aryl" as used herein means a 6-carbons monocyclic or 10-carbons bicyclic aromatic ring system wherein 0, 1, 2, 3, or 4 atoms of each ring are substituted. Examples of aryl groups include phenyl, naphthyl and the like. The term "arylalkyl" or the term "aralkyl" refers to alkyl substituted with an aryl. The term "arylalkoxy" refers to an alkoxy substituted with aryl.

"Cycloalkyl" as used herein means cycloalkyl groups containing from 3 to 8 carbon atoms, such as for example cyclohexyl.

"Heterocyclic" as used herein means heterocyclic groups containing up to 6 carbon atoms together with 1 or 2 heteroatoms which are usually selected from O, N and S, such as for example radicals of : oxirane, oxirene, oxetane, oxete, oxetium, oxalane (tetrahydrofurane), oxole, furane, oxane, pyrane, dioxine, pyranium, oxepane, oxepine, oxocane, oxocinc groups, aziridine, azirine, azirene, azetidine, azetine, azete, azolidine, azoline, azole, azinane, tetrahydropyridine, tetrahydrotetrazine, dihydroazine, azine, azepane, azepine, azocane, dihydroazocine, azocinic groups and thiirane, thiirene, thiethane, thiirene, thietane, thiete, thietium, thiolane, thiole, thiophene, thiane, thiopyrane, thiine, thiinium, thiepane, thiepine, thiocane, thiocinic groups.

"Heterocyclic" may also mean a heterocyclic group fused with a benzene-ring wherein the fused rings contain carbon atoms together with 1 or 2 heteroatom's which are selected from N, O and S.

### INVENTION

Furanic compound of the present invention comprises a furan group that is a group of the heterocyclic aromatic series characterized by a ring structure composed of one oxygen atom and four carbon atoms. The simplest member of the furan family is furan itself

Furanic compound of the invention may comprise at least one hydroxyl function or at least one hydroxyl function. Preferably, the furanic compound comprises at least 2 hydroxyl functions, more preferably 2 hydroxyl functions. Furanic compounds of the present invention may also comprise at least 2 aldehyde functions, more preferably 2 aldehyde functions. The furanic compounds may also for example comprise one hydroxyl function and one aldehyde function.

First reactant is preferably chosen in the group consisting of: furfuryl alcohol, furfural, 2,5-bis(hydroxymethyl)furan, 5-hydroxymethyl furfural (HMF), furan-2,5-dicarbaldehyde and furanose.

5-Hydroxymethylfurfural (HMF) is a biomass-derived compound that can be applied to the synthesis of precursors of pharmaceuticals, furanose-based polymers, monomers of polymers such as polyamide, and other organic intermediates that can lead to numerous chemical products.

Several second reactants of formula (I) may notably be used during the process of the reaction.

R may represent straight, branched or cyclic hydrocarbon group that can be an alkyl, alkenyl, aryl, cycloalkyl or heterocyclic group, eventually comprising one or several heteroatoms such as O, S, F, and N. Preferred groups for R may be for example : H, alkyl, phenyl, benzyl, cycloalkyl, and cycloalkane. R may comprise from 1 to 10 carbon atoms.

Preferred second reactants of the present invention, such as compounds of formula (I), are chosen in the group consisting of: n-heptylamine, methylamine, allylamine, benzylamine, 3-phenylprop-2-enylamine, cyclohexanamine, and (tetrahydrofuran-2-yl)methanamine.

Molar ratio of first reactant to second reactant may be comprised between 1 and 5, preferably between 1 and 2.5.

As previously expressed, a reductant agent may be optionally used in the process of the invention, notably when the furanic compound comprises at least one aldehyde function. Reductant agent, also called reducing agent or reducer, herein refers to an organic or inorganic compound that donates a proton to another species, in a redox reaction. For instance in the reaction of the present invention, reductant agents donate protons to the transiently formed imines. Reductant agents used in the reaction may notably be hydrogen or a secondary alcohol, such as for example isopropanol, glycerol, 2-butanol, and cyclohexanol.

Molar ratio of first reactant to the reductant agent may be comprised between 1 and 10, preferably between 1 and 7.

The furanic compounds comprising at least one amine function are obtained at the end of the reaction. These compounds may comprise one or several primary or secondary amine functions.

The furanic compounds comprising at least one amine function obtained by the process of the present invention are preferably chosen in the group consisting of: (tetrahydrofuran-2,5-diyl) dimethanamine, (furan-2,5-diyl) dimethanamine, 1,6-hexamethylenediamine, 1,1'-(tetrahydrofuran-2,5-diyl)bis(N-methylmethylamine), and 1,1'-(tetrahydrofuran-2,5-diyl)bis(N-heptaneaminomethane).

Preferred reactions of the present invention are the following :
- Reaction of furfuryl alcohol and benzylamine to produce *N-*benzylfurfurylamine
- Reaction of furfural, benzylamine and isopropanol to produce *N-*benzylfurfurylamine
- Reaction of 2,5-bis(hydroxymethyl)furan and benzylamine to produce *N,N*'-bis(benzyl)furan-2,5-diyldimethanamine
- Reaction of HMF, benzylamine and isopropanol to produce *N,N'-*bis(benzyl) furan-2,5- diyldimethanamine

Compound of formula (III) is preferably a (pentamethylcyclopentadienyl)-iridium-ammine iodide, chloride or bromide complex, such as described in R. Kawahara et al. Adv. Synth. Catal. 2011, 353, 1161-1168. [CpIrI₂]₂ and [CpIr(NH₃)₃][I]₂ are particularly preferred as catalysts in the process of the present invention.

Compounds of formula (II) or (III) are preferably water-soluble compounds in the conditions of the reaction, ie homogeneous catalysts.

Molar amount of catalyst of formula (II) or (III) may be comprised between 0.1 and 10 molar %, preferably between 0.5 and 5 molar %, more preferably between 1 and 2 molar %, in relation with the molar amount of first reactant.

The reaction temperature may be comprised between 60 and 150°C. The reaction may be carried out in liquid or gas phase.

The reaction may be performed in the absence or presence of a solvent. Preferred solvents are polar solvents such as water and alcohols.

The reactants, with an optional solvent, and the catalyst are typically combined in a reaction vessel and stirred to constitute the reaction mixture. The reaction mixture is typically maintained at the desired reaction temperature under stirring for a time sufficient to form the furanic compounds comprising amine function(s), in the desired quantity and yield.

The reaction may be carried out in the presence of air but preferably with an inert atmosphere such as N₂ or Ar.

The progress of the reaction toward the furanic compounds comprising at least one amine function may be followed using an appropriate method such as thin layer chromatography, nuclear magnetic resonance, highpressure liquid chromatography, gas chromatography or a combination of the foregoing methods. Exemplary reaction times are 1 to 24 hours, preferably 1 to 8 hours.

The catalyst is typically removed from the reaction mixture using any solid/liquid separation technique such as filtration, centrifugation, and the like or a combination of separation methods. The product may be isolated using standard isolation techniques, such as distillation. At the end of the reaction, the recovery of the compound of formula (II) or (III) from the reaction mixture may be carried out by several known methods such as a phase separation for instance.

The invention is further illustrated by the following non-limiting examples.

### EXPERIMENTAL PART

### Example 1: Production of N-benzylfurfurylamine

To a nitrogen-purged sealed carousel tube was added [Cp*Ir(NH₃)₃][I]₂ (0.02 mol), benzylamine (2.0 mmol), furfuryl alcohol (1.0 mmol), and deionized water (2 mL). The mixture was heated at 100°C under reflux for 6 h and then extracted with ethyl acetate (2 mL X 3). The product was analyzed by GC-MS and ¹H NMR using CH₃CN as reference. *N-*benzylfurfurylamine was obtained in 87% yield with 95% conversion.

### Example 2: Production of N-benzylfurfurylamine

To a nitrogen-purged sealed carousel tube was added [Cp*Ir(NH₃)₃][I]₂ (0.02 mol), benzylamine (2.0 mmol), furfural (1.0 mmol), isopropanol (5 mmol) and deionized water (2 mL). The mixture was heated at 100°C under reflux for 6 h and then extracted with ethyl acetate (2 mL X 3). The product was analyzed by GC-MS and ¹H NMR using CH₃CN as reference. *N*-benzylfurfurylamine was obtained in 84% yield with 98% conversion.

### Example 3: Production of N-heptylfurfurylamine

To a nitrogen-purged sealed carousel tube was added [Cp*Ir(NH₃)₃][I]₂ (0.02 mol), heptylamine (2.0 mmol), furfuryl alcohol (1.0 mmol), isopropanol (5 mmol) and deionized water (2 mL). The mixture was heated at 100°C under reflux for 6 h and then extracted with ethyl acetate (2 mL X 3). The product was analyzed by GC-MS and ¹H NMR using CH₃CN as reference. *N-*heptylfurfurylamine was obtained in 81% yield with 95% conversion.

### Example 4: Production of N,N'-bis(benzyl) furan-2,5-diyldimethanamine

To a nitrogen-purged sealed carousel tube was added [Cp*Ir(NH₃)₃][I]₂ (0.04 mol), benzylamine (2.5 mmol), 2,5-bis(hydroxymethyl)furan (1.0 mmol) and deionized water (2 mL). The mixture was heated at 100°C under reflux for 20 h and then extracted with ethyl acetate (2 mL X 3). The product was analyzed by GC-MS and ¹H NMR using CH₃CN as reference. *N*,*N*'-bis(benzyl) furan-2,5- diyldimethanamine was obtained in 76% yield with 98% conversion.

### Example 5: Production of N,N'-bis(benzyl) furan-2,5-diyldimethanamine

To a nitrogen-purged sealed carousel tube was added [Cp*IrI₂]₂ (0.02 mol), benzylamine (2.5 mmol), HMF (1.0 mmol), isopropanol (5 mmol) and deionized water (2 mL). The mixture was heated at 100°C under reflux for 20 h and then extracted with ethyl acetate (2 mL X 3). The product was analyzed by GC-MS and ¹H NMR using CH₃CN as reference. *N,N'-*bis(benzyl) furan-2,5- diyldimethanamine was obtained in 50% yield with 94% conversion.

## Claims

1. Process for the production of furanic compound comprising at least one amine function, comprising reacting:
- A first reactant being a furanic compound having at least one hydroxyl function or at least one aldehyde function, with
- A second reactant being a compound of formula (I),
R-CH₂-NH₂ (I)
in the presence of a catalyst of formula (II) or (III):
[CpIrX₂]₂ (II)
[CpIr(NH₃)₃][X]₂ (III)
and optionally in the presence of a reductant agent;
wherein:
- R is H or a straight, branched or cyclic hydrocarbon group;
- X is a halide
- Cp is a cyclopentadienyl group, which may be optionally substituted by from 1 to 5 independently selected hydrocarbyl substituents.

2. Process according to claim 1 wherein the first reactant is chosen in the group consisting of: furfuryl alcohol, furfural, 2,5-bis(hydroxymethyl)furan, 5-hydroxymethyl furfural (HMF), furan-2,5-dicarbaldehyde and furanose.

3. Process according to claim 1 or 2 wherein R represents an alkyl, alkenyl, aryl, cycloalkyl or heterocyclic group.

4. Process according to anyone of claims 1 to 3 wherein the second reactant is chosen in the group consisting of: n-heptylamine, methylamine, allylamine, benzylamine, 3-phenylprop-2-enylamine, cyclohexanamine, and (tetrahydrofuran-2-yl)methanamine.

5. Process according to anyone of claims 1 to 4 wherein the molar ratio of first reactant to second reactant is comprised between 1 and 5.

6. Process according to anyone of claims 1 to 5 wherein the reductant agent is hydrogen or a secondary alcohol.

7. Process according to anyone of claims 1 to 6 wherein molar ratio of first reactant to the reductant agent is comprised between 1 and 10.

8. Process according to anyone of claims 1 to 7 wherein the compound of formula (III) is a (pentamethylcyclopentadienyl)-iridium-ammine iodide, chloride or bromide complex.

9. Process according to anyone of claims 1 to 8 wherein molar amount of catalyst of formula (II) or (III) is comprised between 0.1 and 10 molar %, in relation with the molar amount of first reactant.

10. Process according to anyone of claims 1 to 9 wherein the furanic compound comprising at least one amine function is chosen in the group consisting of: (tetrahydrofuran-2,5-diyl) dimethanamine, (furan-2,5-diyl) dimethanamine, 1,6-hexamethylenediamine, 1,1'-(tetrahydrofuran-2,5-diyl)bis(N-methylmethylamine), and 1,1'-(tetrahydrofuran-2,5-diyl)bis(N-heptaneaminomethane).

## Patentansprüche

1. Verfahren zur Herstellung einer Furanverbindung, die mindestens eine Aminfunktion umfasst, umfassend das Umsetzen von:
- einem ersten Reaktanten, der eine Furanverbindung mit mindestens einer Hydroxylfunktion oder mindestens einer Aldehydfunktion ist, mit
- einem zweiten Reaktanten, der eine Verbindung der Formel (I)
R-CH₂-NH₂ (I)
ist, in Gegenwart eines Katalysators der Formel (II) oder (III) :
[CpIrX₂]₂ (II)
[CpIr(NH₃)₃][X]₂ (III)
und gegebenenfalls in Gegenwart eines Reduktionsmittels;
wobei:
- R für H oder eine gerade, verzweigte oder cyclische Kohlenwasserstoffgruppe steht;
- X ein Halogenid ist
- Cp eine Cyclopentadienylgruppe ist, die gegebenenfalls mit 1 bis 5 unabhängig ausgewählten Kohlenwasserstoffsubstituenten substituiert sein kann.

2. Verfahren nach Anspruch 1, wobei der erste Reaktant ausgewählt ist aus der Gruppe bestehend aus: Furfurylalkohol, Furfural, 2,5-Bis(hydroxymethyl)furan, 5-Hydroxymethylfurfural (HMF), Furan-2,5-dicarbaldehyd und Furanose.

3. Verfahren nach Anspruch 1 oder 2, wobei R eine Alkyl-, Alkenyl-, Aryl-, Cycloalkyl oder heterocyclische Gruppe darstellt.

4. Verfahren nach einem der Ansprüche 1 bis 3, wobei der zweite Reaktant ausgewählt ist aus der Gruppe bestehend aus: n-Heptylamin, Methylamin, Allylamin, Benzylamin, 3-Phenylprop-2-enylamin, Cyclohexanamin und (Tetrahydrofuran-2-yl)methanamin.

5. Verfahren nach einem der Ansprüche 1 bis 4, wobei das Molverhältnis von erstem Reaktanten zu zweitem Reaktanden zwischen 1 und 5 liegt.

6. Verfahren nach einem der Ansprüche 1 bis 5, wobei das Reduktionsmittel Wasserstoff oder ein sekundärer Alkohol ist.

7. Verfahren nach einem der Ansprüche 1 bis 6, wobei das Molverhältnis des ersten Reaktanten zu dem Reduktionsmittel zwischen 1 und 10 liegt.

8. Verfahren nach einem der Ansprüche 1 bis 7, wobei die Verbindung der Formel (III) ein (Pentamethylcyclopentadienyl)-iridiumamminiodid-, - chlorid oder -bromidkomplex ist.

9. Verfahren nach einem der Ansprüche 1 bis 8, wobei die molare Menge des Katalysators der Formel (II) oder (111) zwischen 0,1 und 10 Mol-%, bezogen auf die molare Menge des ersten Reaktanten, beträgt.

10. Verfahren nach einem der Ansprüche 1 bis 9, wobei die Furanverbindung, die mindestens eine Aminfunktion umfasst, ausgewählt ist aus der Gruppe bestehend aus: (Tetrahydrofuran-2,5-diyl)dimethanamin, (Furan-2,5-diyl)dimethanamin, 1,6-Hexamethylendiamin, 1,1'-(Tetrahydrofuran-2,5-diyl)bis(N-methylmethylamin) und 1,1'-Tetrahydrofuran-2,5-diyl)bis(N-heptanaminomethan).

## Revendications

1. Procédé de production de composé furanique comprenant au moins une fonction amine, comprenant la réaction de :
- un premier réactif étant un composé furanique ayant au moins une fonction hydroxyle ou au moins une fonction aldéhyde, avec
- un deuxième réactif étant un composé de formule (I),
R-CH₂-NH₂ (I)
en présence d'un catalyseur de formule (II) ou (III) :
[CpIrX₂]₂ (II)
[CpIr(NH₃)₃][X]₂ (III)
et facultativement en présence d'un agent réducteur ; dans lequel :
- R est H ou un groupe hydrocarboné linéaire, ramifié ou cyclique ;
- X est un halogénure
- Cp est un groupe cyclopentadiényle, qui peut être facultativement substitué par 1 à 5 substituants hydrocarbyle indépendamment choisis.

2. Procédé selon la revendication 1 dans lequel le premier réactif est choisi dans le groupe constitué de : l'alcool furfurylique, le furfural, le 2,5-bis(hydroxyméthyl)furane, le 5-hydroxyméthyl-furfural (HMF), le furane-2,5-dicarbaldéhyde et le furanose.

3. Procédé selon la revendication 1 ou 2 dans lequel R représente un groupe alkyle, alcényle, aryle, cycloalkyle ou hétérocyclique.

4. Procédé selon l'une quelconque des revendications 1 à 3 dans lequel le deuxième réactif est choisi dans le groupe constitué des : n-heptylamine, méthylamine, allylamine, benzylamine, 3-phénylprop-2-énylamine, cyclohexanamine et (tétrahydrofurane-2-yl)méthanamine.

5. Procédé selon l'une quelconque des revendications 1 à 4 dans lequel le rapport molaire du premier réactif au deuxième réactif est compris entre 1 et 5.

6. Procédé selon l'une quelconque des revendications 1 à 5 dans lequel l'agent réducteur est hydrogène ou un alcool secondaire.

7. Procédé selon l'une quelconque des revendications 1 à 6 dans lequel le rapport molaire du premier réactif à l'agent réducteur est compris entre 1 et 10.

8. Procédé selon l'une quelconque des revendications 1 à 7 dans lequel le composé de formule (III) est un complexe d'iodure, chlorure ou bromure de (pentaméthylcyclopentadiényl)-iridium-amine.

9. Procédé selon l'une quelconque des revendications 1 à 8 dans lequel la quantité molaire de catalyseur de formule (II) ou (III) est comprise entre 0.1 et 10 % en moles, par rapport à la quantité molaire du premier réactif.

10. Procédé selon l'une quelconque des revendications 1 à 9 dans lequel le composé furanique comprenant au moins une fonction amine est choisi dans le groupe constitué des : (tétrahydrofurane-2,5-diyl)diméthanamine, (furane-2,5-diyl)diméthanamine, 1,6-hexaméthylènediamine, 1,1'-(tétrahydrofurane-2,5-diyl)bis(N-méthylméthylamine) et 1,1'-(tétrahydrofurane-2,5-diyl)bis(N-heptaneaminométhane).
